(19) Europäisches Patentamt / European Patent Office / Office européen des brevets

(11) **EP 2 323 731 B1**

(12) **EUROPEAN PATENT SPECIFICATION**

(45) Date of publication and mention
of the grant of the patent:
**08.10.2014 Bulletin 2014/41**

(51) Int Cl.:
*A61N 1/37* *(2006.01)*     *A61B 5/053* *(2006.01)*
*A61B 5/00* *(2006.01)*     *A61N 1/362* *(2006.01)*
*A61N 1/365* *(2006.01)*

(21) Application number: **08794115.9**

(22) Date of filing: **29.08.2008**

(86) International application number:
**PCT/SE2008/000489**

(87) International publication number:
**WO 2010/024738 (04.03.2010 Gazette 2010/09)**

(54) **IMPLANTABLE MEDICAL DEVICE AND METHOD FOR SUCH A DEVICE FOR PREDICTING HF STATUS OF A PATIENT**

IMPLANTIERBARE MEDIZINISCHE VORRICHTUNG UND VERFAHREN FÜR EINE SOLCHE VORRICHTUNG ZUR VORHERSAGE DES HF-STATUS EINES PATIENTEN

DISPOSITIF MÉDICAL IMPLANTABLE ET PROCÉDÉ POUR QU'UN TEL DISPOSITIF PRÉDISE L'ÉTAT D'INSUFFISANCE CARDIAQUE D'UN PATIENT

(84) Designated Contracting States:
**AT BE BG CH CY CZ DE DK EE ES FI FR GB GR HR HU IE IS IT LI LT LU LV MC MT NL NO PL PT RO SE SI SK TR**

(43) Date of publication of application:
**25.05.2011 Bulletin 2011/21**

(73) Proprietor: **St. Jude Medical AB**
**175 84 Järfälla (SE)**

(72) Inventors:
• **BJÖRLING, Anders**
**S-169 37 Solna (SE)**
• **PANESCU, Dorin**
**San Jose**
**CA 95136 (US)**
• **ZHAO, Wenxia**
**Thousand Oaks**
**CA 91320 (US)**

(74) Representative: **Sjölander, Henrik et al**
**Aros Patent AB**
**P.O. Box 1544**
**751 45 Uppsala (SE)**

(56) References cited:
WO-A2-03/041797     US-A1- 2005 215 914
US-A1- 2007 142 732     US-A1- 2008 024 293
US-B1- 6 512 949     US-B1- 6 741 885
US-B1- 7 308 309

• **FUNG J. W-H ET AL.: 'Implantable cardiac resynchronization therapy devices to monitor heart failure clinical status' CURRENT HEART FAILURE REPORTS vol. 4, no. 1, 2007, pages 48 - 52, XP003024295**

## Description

## Technical field

The present invention generally relates to implantable medical devices, such as pacemakers or implantable cardioverter/defibrillators (ICDs), and in particular to techniques for detecting and predicting changes of heart conditions, such as heart failure, at an early stage within a patient in which a medical device is implanted.

## Background of the invention

Heart failure is a debilitating disease in which abnormal function of the heart may lead to inadequate blood flow to fulfil the needs of tissues and organs of the body. Typically, the heart loses propulsive power because the cardiac muscle loses capacity to stretch and contract. Often, the ventricles do not adequately eject or fill with blood between heartbeats and the valves regulating blood flow become leaky, allowing regurgitation or back-flow of blood. The impairment of arterial circulation deprives vital organs of oxygen and nutrients. Fatigue, weakness and the inability to carry out daily tasks may result. Not all heart failure patients suffer debilitating symptoms immediately and some may live actively for years. Yet, with few exceptions, the disease is relentlessly progressive. As heart failure progresses, it tends to be increasingly difficult to manage. A particularly severe form of heart failure is congestive heart failure (CHF) wherein the weak pumping of the heart leads to build-up of fluid in the lungs and other organs and tissues.

New medical and device-based therapies for heart failure improve survival and reduce hospitalization rates. However, total hospitalization continues to rise with the growing prevalence of heart failure. In fact, only in Europe approximately 14 million people suffer from heart failure and about 3.6 million new cases are diagnosed every year. The 5 year mortality is approximately 50%. Consequently, treating HF poses a huge stress on all European countries' as well as USA's economic systems and, for example, in United States heart failure is one of the most costly diseases in healthcare budgets, with 70 % of the expenses going to the treatment of acute heart failure de-compensation.

Patients with HF eventually experiences episodes of HF exacerbation requiring some kind of intervention - in some cases even hospitalization. These exacerbations may be triggered by a number of factors such as non-compliance to drugs, development of AF, changes in diet or alcohol intake, influenza etc. but may also be idiopathic. By predicting these exacerbations or more closely following the patient's HF status, early intervention is possible which may prevent the need for costly hospitalization. This also improves patient care, and in the long run, may even improve survival. Although regular monitoring of heart failure patients is recommended in management programs, none of these measures has shown conclusive impact on heart failure morbidity.

Symptoms leading to heart failure hospitalization usually occur late in the course of de-compensation. Therefore, a reliable means of chronic fluid status monitoring in HF patients is needed to detect early de-compensation when appropriate intervention is possible.

Both literature and clinical and pre-clinical work have shown that impedance, both intracardiac and intrathoracic, decrease as HF progresses and drops significantly prior to an acute HF exacerbation.

For example, US 2008/0024293 described an algorithm that assesses the patients' HF status using the impedance. A reference value or impedance is established as the average over a period of three days. This reference impedance is adapted over time to the measured impedances, increasing or decreasing daily by a small amount in order to approximate the patients' long-term impedance trend. On days when the measured impedance is less than the reference impedance, the difference between the measured impedance and the reference impedance is cumulative summed to produce a fluid index. If the measured impedance is significantly greater than the reference impedance, the fluid index is set to 0. The fluid index is compared to a threshold to detect a sustained transient decrease in impedance.

In U.S. Pat Appl. No. 2006/0276848, a method using impedance measures to monitor patient condition is shown. Impedance values are acquired over one or more respiratory cycles and an impedance span is determined based on the impedance values. A cardiac condition is determined using the impedance span. Recently acquired impedance information can be compared to historic information to determine the patient condition.

Furthermore, in U.S. Pat. No. 7,272,443, a method for predicting the onset of a heart condition such as heart failure is disclosed. Impedance values representative of thoracic fluid levels within the patient and impedance values representative of ventricular mass are detected and the onset is then predicted based on thoracic fluid levels in combination with ventricular mass. Changes in thoracic fluid levels are monitored to detect a fluid overload, i.e. a significant increase in thoracic fluid levels.

Accordingly, great efforts have been made within the art to develop methods for predicting HF worsening on an early stage. However, even if the prior art methods generally provides reasonable accuracy and reliability, problems related to so called false positive detections, which are stressing for the patient as well as for the health care providers, are often encountered. This entails a remaining need within the art for improved methods and, in particular, there is an interest within the art of reducing the number of false- positive detections without impairing the accuracy of the prediction.

Thus, it remains a need within the art for improved devices and methods for an accurate and reliable prediction of HF worsening on an early stage at the same time as the number or amount of false-positive detections can be reduced.

## Summary of the invention

Thus, an object of the present invention is to provide an improved medical device for predicting HF worsening at an early stage.
A further object is to provide an improved medical device that is capable of reducing the number of false positive indications of HF status worsening.

Yet another object of the present invention is to provide an improved medical device that is capable of, in an accurate and reliable manner, predicting HF worsening at an early stage at the same time as the number or amount of false-positive detections of HF worsening can be reduced.

These and other objects of the present invention are achieved by means of an implantable medical device having the features defined in the independent claims. Preferable embodiments of the invention are characterized by the dependent claims.

According to a first aspect of the present invention, there is provided an implantable medical device for monitoring a patient status, for example, for monitoring HF status and predicting a worsening of the HF status of the patient. The medical device comprises an impedance circuit adapted to acquire impedance signals during consecutive impedance measuring sessions, an impedance processing module adapted to process the impedance signals to determine a first trend parameter based on multiple impedance signals measured over a first period of time and to determine a second trend parameter based on multiple impedance signals measured over a second period of time, which second period of time is significantly longer than the first period of time, and a patient status determining module adapted to determine a patient status index, wherein the first trend parameter and the second trend parameter are compared at predetermined sample points of time. Further, the patient status determining module is adapted to count samples, when impedance trend parameters are used, at which the first trend parameter is lower than the second trend parameter or to count samples, when admittance trend parameters are used, at which the first trend parameter is higher than the second trend parameter. A sample may be counted if the ratio or quote between the trend parameters is outside predetermined upper and lower limits. Thereby, it is possible to filter out small changes that may be caused by, for example, posture changes of the patient.

In one embodiment, the patient status index corresponds to the counts and where the index is increased for each count. Moreover, the patient status determining module is adapted to determine a patient status based on the patient status index, wherein a patient status index that has increased substantially monotonously during a first monitoring period is determined to be an indication of an exacerbation of patient status, and in particular, a worsening of heart failure.

In the context of the present invention, the term "substantially monotonously" is to be regarded as an increase (or decrease) that has continued almost continuously for a period of time, possibly interrupted by short periods of status quo (i.e. no change of the observed signal) or short periods of a movement in the opposite direction, provided that these short periods are significantly shorter than the period of time over which the monotonous increase or decrease has lasted. For example, in one embodiment of the present invention, the period over which the monotonous increase or decrease is observed is about 11 days and the short periods of status quo or movement in the opposite direction allowed to take place as interruptions of the monotonous increase or decrease is about 2 days.

According to a second aspect of the present invention, there is provided an implantable medical device for monitoring a patient status, comprising an impedance circuit adapted to acquire impedance signals during consecutive impedance measuring sessions; an impedance processing module adapted to process the impedance signals to determine a first trend parameter based on multiple impedance signals measured over a first period of time and to determine a second trend parameter based on multiple impedance signals measured over a second period of time, which second period of time is significantly longer than the first period of time, wherein the impedance processing module is adapted to calculate each impedance value as a composite value of impedance values acquired by means of at least two impedance measurement vector configurations, each impedance configuration being associated with a predetermined weight factor. Further, the implantable device includes a patient status determining module adapted to: determine a patient status index, wherein the first trend and the second trend is compared at predetermined sample points of time; and determine a patient status based on the patient status index, wherein an indication of an exacerbation of the patient status is determined if predetermined conditions are satisfied.

There is provided a method for monitoring a patient status, for example, for monitoring a HF status of the patient or predicting a worsening of HF status of the patient. The method includes acquiring impedance signals during consecutive impedance measuring sessions, storing the acquired impedance signals, processing the impedance signals to determine a first trend parameter based on multiple impedance signals measured over a first period of time and to determine a second trend parameter based on multiple impedance signals measured over a second period of time, which second period of time is significantly longer than the first period of time, determining a patient status index, including comparing the first trend parameter and the second trend parameter at predetermined sample points of time, counting samples, when impedance trend parameters are used, at which the first trend parameter is lower than the second trend parameter or samples, when admittance trend parameters are used, at which the first trend parameter is higher than the second trend parameter, determining the patient status index to correspond to the counts, and determining a patient status based on the patient status index, wherein a patient status index that has increased substantially monotonously during a first monitoring period is determined to be an indication of an exacerbation of patient status and, in particular, a worsening of the HF status.

There is provided a method for monitoring a patient status, comprising: acquiring impedance signals during consecutive impedance measuring sessions; processing the impedance signals to determine a first trend parameter based on multiple impedance signals measured over a first period of time and to determine a second trend parameter based on multiple impedance signals measured over a second period of time, which second period of time is significantly longer than the first period of time; calculating each impedance value as a composite value of impedance values acquired by means of at least two impedance measurement vector configurations, wherein each impedance configuration is associated with a predetermined weight factor; determining a patient status index, including comparing the first trend parameter and the second trend parameter at predetermined sample points of time; and determining a patient status based on the patient status index, wherein an indication of an exacerbation of the patient status is determined if predetermined conditions are satisfied.

In embodiments of the present invention, the trend parameter is impedance or admittance averages. However, as will be discussed below, sums of the impedances or admittances may be used as an alternative.

A basic idea of the invention is to utilize impedance data to evaluate and predict a patient's HF (heart failure) status. It has been shown, in both literature and in clinical studies as well as in pre-clinical work, that impedance, both intracardiac and intrathoracic, decrease as HF progresses and drops significantly prior to an acute HF exacerbation. The present invention utilizes this fact and aims at capturing the impedance drops at an early stage to track the patient's HF status and to predict the HF worsening. Impedance or admittance information is hence collected or acquired in order to track the HF status of the patient and the impedance or admittance information is processed to determine a long term trend parameter and a short term trend parameter over time, where the period of time used in the short term trend parameter is significantly shorter than the period of time used in the long term trend parameter. By comparing the short term trend parameter with the long term trend parameter it is possible to gain insight of the trend of the impedance or admittance change over time, and, hence, gain insight in the changes of the HF status over time. A patient status index is determined that reflects the ratio between short term trend parameter and the long term trend parameter and, more specifically, the number of times the short term trend parameter is lower than the long term trend parameter is counted, when impedance trend parameters are used, or the number of times the long term trend parameter is below the short term trend parameter is counted when admittance trend parameters are used. In particular, it has been observed by the inventors that if the impedance has decreased or the admittance has increased substantially monotonously over a predetermined period of time, which means that the short term trend parameter mostly (i.e. at the majority of the counts, the short term trend parameter has been lower than the long term trend parameter) has been lower than the long term trend parameter during the predetermined period of time, this is an indication of a worsening of the HF status of the patient.

The present invention is advantageous for a number of reasons. First of all, it has been shown that the use of a long term trend parameter of the impedance or admittance calculated over a period of time being significantly longer than a period of time used to calculate a short term trend parameter of the impedance or admittance and that to base a patient status index on a qualitative comparison of these two trend parameters provides valuable information of the HF status and especially on the changes of the HF status. Above all, it has been shown that such a patient status index that discloses a substantially monotonous increase during a predetermined period of time is an early indication of a worsening or exacerbation of the HF status of the patient. Hence, the present invention provides means for predicting a worsening or exacerbation of the HF status at an early stage thereby improving patient care. This also entails that an early intervention is possible if required which may prevent the need for costly hospitalization. Furthermore, the present invention also enables a reduction of the number of false positives - a false indication of a worsening of the HF status - due to the facts that:

- the period used for the long term trend parameter is significantly longer than the period used for the short term trend parameter,
- the trend parameters, the long term and the short term, are evaluated over a predetermined period of time, and
- the criteria for an indication of a worsening of the HF status is set to a substantially monotonous increase over the predetermined period.

Thereby, the patient will not be unnecessarily worried and the health care providers will not be burdened by unnecessary false alarms of acute HF episodes from HF patients.

According to embodiments of the present invention, patient specific thresholds are used. For example, the change of the patient status index that indicates a worsening of heart failure is patient specific. Thereby, the change of the patient status index can be individually adjusted to specific patient conditions. For example, for one patient a substantially monotonous increase during a period of 11 days or 264 hours may be an indication of a worsening of heart failure and allowed interruptions might be set to about 1 day or 24 hours. For another patient, a substantially monotonous increase during a period of 9 days or 216 hours may be an indication of a worsening of heart failure and allowed interruptions might be set to about half a day or 12 hours. Further, the first trend parameter is compared with the second trend parameter and each sample where the first trend parameter is lower than the second trend parameter is counted and the amount with which the first trend parameter has to be lower than the second trend parameter in order to be counted may be patient specific. By such patient specific threshold, the monitoring and prediction can be made more accurate and reliable.

According to one embodiment of the present invention, in connection with the first aspect of the invention, some or each impedance value used to determine the first and/or second trend parameters is calculated as a composite value of impedance values acquired by means of at least two impedance measurement vector configurations, wherein each impedance configuration is associated with a predetermined weight factor.

According to another embodiment of the present invention, in connection with the second aspect of the invention, samples are counted, when impedance trend parameters are used, at which the first trend parameter is lower than the second trend parameter, or samples are counted, when admittance trend parameters are used, at which the first trend parameter is higher than the second trend parameter, and wherein the patient status index corresponds to the counts; and a patient status is determined based on the patient status index, wherein the predetermined conditions includes that a patient status index that has increased substantially monotonously during a first monitoring period is determined to be an indication of an exacerbation of patient status.

In one embodiment of the present invention, samples are counted, when impedance trend parameters are used, at which the first trend parameter is lower or above than the second trend parameter, wherein the index is increased at a counted sample when the first trend parameter is lower than the second trend parameter and decreased when the first trend parameter is above the second parameter, samples are counted, when admittance trend parameters are used, at which the first trend parameter is higher or lower than the second trend parameter, and wherein the patient status index corresponds to the counts, wherein the index is decreased at a counted sample when the first trend parameter is lower than the second trend parameter and increased when the first trend parameter is above the second parameter; and a patient status is determined based on the patient status index, wherein the predetermined conditions includes that a patient status index that has increased substantially monotonously during a first monitoring period is determined to be an indication of an exacerbation of patient status.

In embodiments of the present invention, the short term trend parameter is calculated as a moving trend parameter over a first period of time. A preferred period of time is between about 0-5 days, another preferred period of time is about 1-3 days, a further preferred period of time is about 12 and about 48 hours, a more preferred period of time is about 18-42 hours, an even more preferred period of time is about 18-36 hours, and highly preferred period of time is about 24 hours. Further, the long trend parameter is calculated as a moving trend parameter over a second period of time. A preferred period of time is between about 7-75 days, another preferred period of time is about 10-45 days, a further preferred period of time is about 10-30, yet another preferred period of time is about 12-25 days, a more preferred period of time is about 12-18 days, an even more preferred period of time is about 12-16 days, and highly preferred period of time is about 14 days.

In one embodiment of the present invention, a situation where the patient status index has increased such that a predetermined threshold has been exceeded during the first monitoring period is determined to be an indication of an exacerbation of the patient status.

In one embodiment of the present invention, a sample may be counted if the ratio or quote between the trend parameters is outside predetermined upper and lower limits. Thereby, it is possible to filter out small changes that may be caused by, for example, posture changes of the patient. Further, as discussed above, the limits may also be patient specific.

According to an embodiment of the present invention, each counted sample is associated with predetermined weight in accordance with a time interval to a preceding sample. For example, if the sample interval is changed from two hours to one hour, the weight for the two-hour interval may be set to 1 while the weight for the one-hour interval is set to 0.5. Thereby, it is assured that a change of sample interval not will affect the patient status index in an unduly way. It might be of interest to have a smaller sample interval if, for example, the patient status index has increased substantially monotonously during a certain monitoring period, which is shorter than the predetermined period of time which is a criteria for an indication of a worsening of the HF status.

According to a further embodiment of the present invention, samples, when impedance trend parameters are used, at which the first trend parameter is higher than the second trend parameter are counted or samples, when admittance trend parameters are used, at which the first trend parameter is lower than the second trend parameter are counted. Based on these counts a reset measure is determined and the patient status index is reset to an initial value if the reset measure has increased substantially monotonously during a second monitoring period, the second monitoring period being substantially shorter than the first monitoring period. Thereby, the number of false positive indications can be reduced significantly due to the fact the patient index is reset if an improvement of the HF status is detected. This second monitoring periods is set to be about 0-5 days in one embodiment, about 1-3 days in preferred embodiments of the present invention and about two days in another preferred embodiment of the present invention.

In a further embodiment of the present invention, the patient status index is reset to the initial value if the reset measure has increased such that a predetermined reset threshold has been exceeded during the second monitoring period.

According to one embodiment of the present invention, a temporary value reflecting a ratio between the first trend parameter and the second trend parameter is determined and if, when impedance trend parameters are used, the temporary value is below a predetermined value or if, when admittance trend parameters are used, the temporary value above a predetermined value, a sum for a present sample to be equal to the sum at a preceding sample and the temporary value multiplied with the period of time lapsed since the preceding sample is determined, wherein the patient status index is determined to correspond to the sum for the present sample. Thus, the magnitude of the impedance drop or decrease is taken account of as well as the time the decrease has lasted. Thereby, the reliability and accuracy of the prediction can be further improved and number of false indications even further reduced. The temporary value may be determined as being equal to the quote between the first trend parameter and the second trend parameter subtracted with a value corresponding to 1; and the temporary value is also rectified.

In an alternative embodiment, if processing power is of importance, the long average in the previous embodiments could be replaced by the sum of the impedance or admittance values during the long window and the short average could be replaced by the sum of the impedance or admittance values within the short window, multiplied with the quotient of the time of the long and short windows. By doing so, the divisions needed to calculate the two averages are replaced by a single multiplication, which is less power consuming to realize in a device. Furthermore, the long and short windows could be set so the quotient between them equals 2 raised to an integer, i.e. 2, 4, 8, 16 etc. Thus, the multiplication may in turn be replaced by a shift operation, which is even less power consuming.

In an alternative embodiment, a temporary value reflecting a ratio between the second trend parameter and the first trend parameter is determined and if the temporary value exceeds a predetermined value when impedance trend parameters are used or below the predetermined value when admittance trend parameters are used a sum for a present sample is determined to be equal to the sum at a preceding sample and the ratio multiplied with the period of time elapsed since the preceding sample, wherein the patient status index is determined to correspond to the sum for the present sample.

The temporary value may be determined as being equal to the quote between the second trend parameter and the first trend parameter subtracted with a value corresponding to 1.

According to embodiments of the present invention, each impedance value used to determined the trend parameters is calculated as a composite value of a number of impedance values, each corresponding to an impedance signal acquired by means of a specific impedance measurement vector configuration, wherein each impedance configuration is associated with a predetermined weight factor. An arbitrary number of measurement vector may be used for each impedance value. For example, the following formula may be used:

$$Z = \mathit{offset} + \sum_{i=1}^{N} w_i \times Z_i \cdot \frac{1}{N}$$

where N is the number of impedance configurations, $w_i$, the weight factor for impedance configuration i and $Z_i$ the impedance value for configuration i. The weight factors , $w_i$, could, for example, be set to one over the standard deviation of the respective impedance configuration values during a reference period. Thereby, each of the impedance configurations response to a changed physiological situation will be similar in size, i.e. each vector will affect $Z$ the same amount. Similarly, the admittance signal can be calculated as:

$$Y = \mathit{offset}' + \sum_{i=1}^{N} w_i' \times Y_i \cdot \frac{1}{N}$$

where $w_i'$, the weight factor, may be one over the standard deviation of the admittance values for the N vectors, respectively.

In accordance with a further embodiment of the present invention, the patient status index is compared with a predetermined patient specific threshold and, if the patient status index exceeds the patient specific threshold, a warning signal that triggers an alarm function is issued. Preferably, the threshold is programmable. The threshold will be different for different methods of determining the patient status index and for predicting the HF status and will also be patient specific. Since the impedance is affected not only by the HF status but also be lead location, blood constituents, if the patient is on dialysis, diet, exercise, posture, etc, the impedance (and hence the index) variability will be different for different patients. The alarm may be sent to the physician of the patient via a communication system using a system for remote follow-up. The physician may call the patient for a visit, or the device may notify the patient that he or she should seek contact with the/a physician.

According to an embodiment of the present invention, the patient data is transferred to and displayed on an external device such as programmer during a follow-up and thus a trend of the patient's HF status can thus be reviewed by, for example, a physician.

In a further embodiment of the present invention, the patient status is regularly transferred to and displayed on an external device such as a home monitoring device located at the patient's home, and/or a programmer device located at the health care provider of the patient.

As the skilled person realizes, steps of the methods, as well as preferred embodiments thereof, are suitable to realize as computer program or as a computer readable medium.

Further objects and advantages of the present invention will be discussed below by means of exemplifying embodiments.

**Brief description of the drawings**

Exemplifying embodiments of the invention will be described below with reference to the accompanying drawings, in which:

Fig. 1 is a simplified, partly cutaway view, illustrating an implantable medical device according to the present invention with a set of leads implanted into the heart of a patient;
Fig. 2 is a functional block diagram form of the implantable medical device shown in Fig. 1, illustrating basic circuit elements that provide, for example, pacing stimulation in the heart and particularly illustrating components for predicting heart failure;
Fig. 3 is a flow chart describing the general principles of the method according to an embodiment;
Fig. 4 is a functional block diagram form of a system environment in which the present invention can be implemented;
Fig. 5 shows in the upper graph the impedance, the raw, the short term trend parameter and the long term trend parameter, measured for one patient over a period of time and the corresponding patient status index in the lower graph, wherein the patient status index is determined in accordance with a first embodiment of the present invention; and
Fig. 6 shows in the upper graph the impedance, the raw, the short term trend parameter and the long term trend parameter, measured for another patient over another period of time and the corresponding patient status index in the lower graph, wherein the patient status index is determined in accordance with another embodiment of the present invention.

**Description of exemplifying embodiments**

The following is a description of exemplifying embodiments in accordance with the present invention. This description is not to be taken in limiting sense, but is made merely for the purposes of describing the general principles of the invention. Thus, even though particular types of implantable medical devices such as heart stimulators will be described, e.g. biventricular pacemakers, the invention is also applicable to other types of cardiac stimulators such as dual chamber stimulators, implantable cardioverter defibrillators (ICDs), etc.

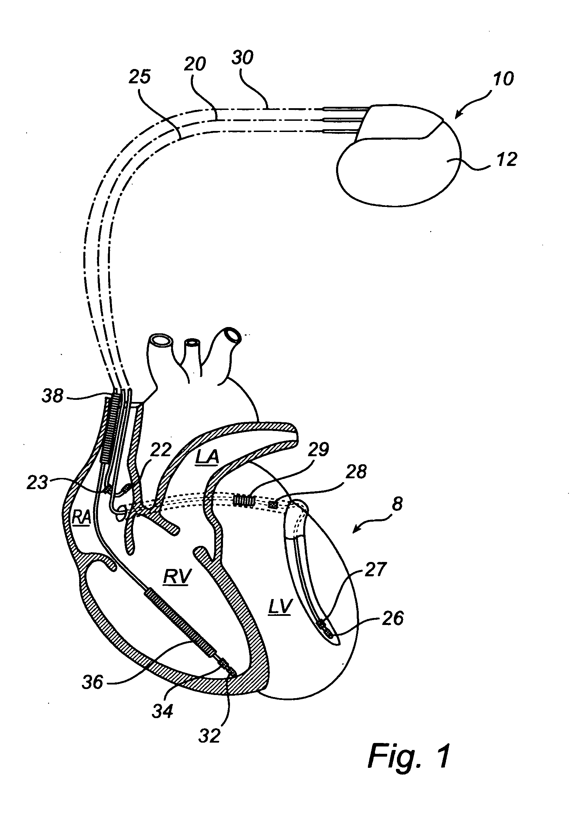

With reference first to Fig. 1, there is shown an implantable medical device capable of predicting heart failure according to an embodiment of the present invention. According to this embodiment, the invention is implemented in a stimulation device 10 having a case 12. The stimulation device 10 is in electrical communication with a patient's heart 1 by way of a left atrial lead 20 having a right atrial (RA) tip electrode 22 and a RA ring electrode 23 implanted in the atrial appendage. Further, the stimulator 10 is also in electrical communication with the heart 1 by way a right ventricular lead 30 having a right ventricular (RV) tip electrode 32, a RV ring electrode 34, RV coil electrode 36, and a superior vena cava (SVC) coil electrode 38. Typically, the RV lead is transvenously inserted into the heart 1 so as to place the RV coil electrode 36 in the right ventricular apex and the SVC coil electrode 38 in the superior vena cava. Accordingly, the right ventricular lead 30 is capable of receiving cardiac signals, and delivering stimulation in the form of pacing to the right ventricle RV.

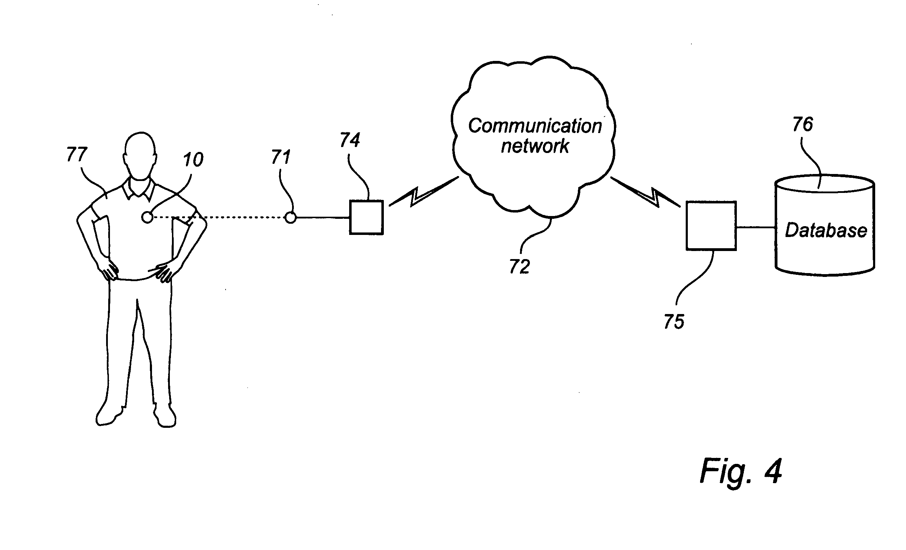

In order to sense left atrial and ventricular cardiac signals and to provide left chamber pacing therapy, the stimulator 10 is coupled to a "coronary sinus" lead 25 designed for placement in the coronary sinus region via the coronary sinus for positioning a distal electrode adjacent to the left atrium. As used herein, the wording "coronary sinus region" refers to the vasculature of the left ventricle, including any portion of the coronary sinus, great cardiac vein, left marginal vein, middle cardiac vein, and/or small cardiac vein or any other cardiac vein accessible by the coronary sinus. Accordingly, the coronary sinus lead 25 is designed to receive atrial and ventricular pacing signals and to deliver left ventricular pacing therapy using at least a left ventricular (LV) tip electrode 26, a LV ring electrode 27 left atrial pacing therapy using at least a LA coil electrode 28 and a LA coil electrode 29. With this configuration biventricular therapy can be performed. Although only three medical leads are shown in Fig. 4, it should also be understood that additional stimulation leads (with one or more pacing, sensing, and/or shocking electrodes) may be used in order to efficiently and effectively provide pacing stimulation to the left side of the heart or atrial cardioversion.

With reference to the configuration shown in Fig. 1, a number of impedances vectors that can be used for impedance measurements, for example, between the RV coil electrode 36 and the case 12, LV ring electrode 27 and the RA ring electrode 23, the RV ring electrode 34 and the LV ring electrode 27, the LV ring electrode 27 and the case 12, the RA ring electrode 23 and the case 12, and the RV ring electrode 27 and the case 12. Although these configurations have been shown to provide good measurement results, it is understood that other configurations can be used and that the present invention is not limited to these six configurations. For example, measurements vectors including more than two electrodes can be used including three or four electrodes so called tripolar or quadropolar, respectively, vectors.

Thus, in accordance with the present invention impedance or admittance signals are acquired by means of, for example, any one of the above-mentioned bipolar measurement vectors by applying a current of known amplitude, the current is preferably applied in pulse chains, between the two electrodes and then measuring the resulting voltage between the electrodes. The impedance, Z, is calculated by taking the quotient of the resulting voltage, U, and the applied current, I:

$$Z = U/I.$$

The admittance is calculated by inverting this quotient:

$$Y = 1/Z = I/U.$$

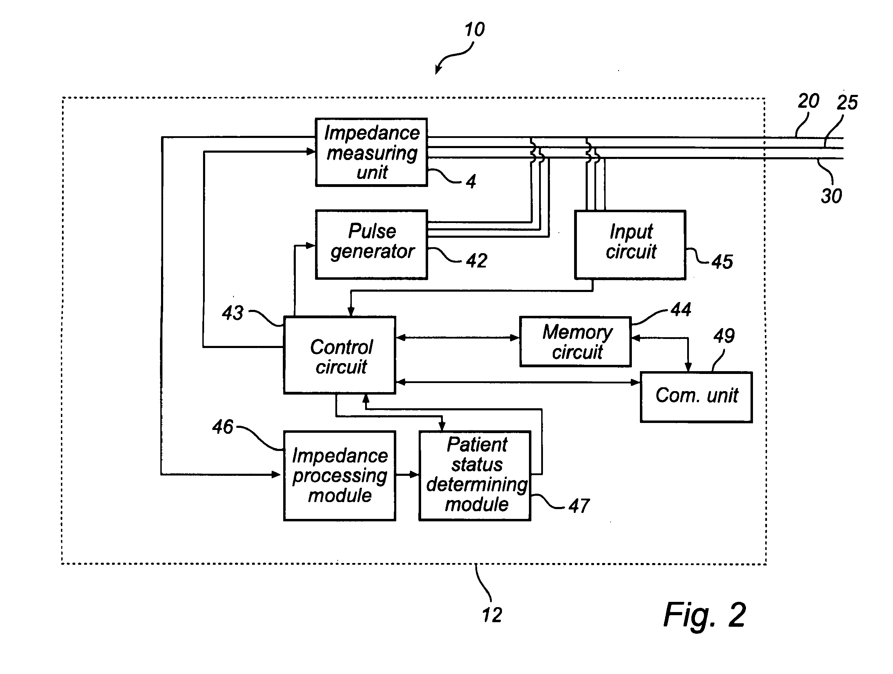

A simplified block diagram showing the internal components of the stimulator 10 is shown in Fig. 2.

Turning now to Fig. 2, the heart stimulator 10 of Fig. 1 is shown in a block diagram form. For illustrative purposes, reference is made to Fig. 1 for the elements of the leads that are intended for positioning in or at the heart. The heart stimulator 10 comprises a housing 12 being hermetically sealed and biologically inert, see Fig. 1. Normally, the housing is conductive and may, thus, serve as an electrode. One or more pacemaker leads, where three are shown in Fig. 1, 20, 25 and 30, are electrically coupled to the implantable medical device 10 in a conventional manner. The leads 20, 25, and 30 extend into the heart (see Fig. 1).

As discussed above with reference to Fig. 1, the leads 20, 25, and 30 comprises one or more electrodes, such a coils, tip electrodes or ring electrodes, arranged to, inter alia, transmit pacing pulses for causing depolarization of cardiac tissue adjacent to the electrode(-s) generated by a pace pulse generator 42 under influence of a control circuit 43 comprising a microprocessor and for measuring impedances reflecting the septal wall movements. The control circuit 43 controls, inter alia, pace pulse parameters such as output voltage and pulse duration. A memory circuit 44 is connected to the control circuit 43, which memory circuit 44 may include a random access memory (RAM) and/or a non-volatile memory such as a read-only memory (ROM). Detected signals from the patients heart are processed in an input circuit 45 and are forwarded to the microprocessor of the control circuit 43 for use in logic timing determination in known manner. The stimulator also includes a communication unit 49, for example, a telemetry unit or RF transceiver adapted for bi-directional communication with external devices.

Furthermore, an impedance measuring unit 41 is adapted to carry out impedance measurements of the intracardiac and intrathoracic impedance or admittance of the patient, for example, by means of applying a current over any one, some of, or all of the following measurements vectors between the RV coil electrode 36 and the case 12, LV ring electrode 27 and the RA ring electrode 23, the RV ring electrode 34 and the LV ring electrode 27, the LV ring electrode 27 and the case 12, the RA ring electrode 23 and the case 12, and the RV ring electrode 27 and the case 12. The resulting voltage is, according to these examples, measured between the same electrodes. The raw impedance data is then supplied to an impedance processing module 46, which may comprise, for example, amplifiers and filters e.g. FIR or IIR filters. The impedance processing module 46 performs a pre-processing procedure in which the acquired impedance or admittance signal are pre-processed. For example, the impedance can be calculated according to the following formula:

$$Z_{new} = offset + \sum_{i=1}^{N} w_i \times Z_i \cdot \frac{1}{N}$$

where N is the number of impedance configurations, $w_i$, the weight factor for impedance configuration i and $Z_i$ the impedance value for configuration i. The weight factors , $w_i$, could, for example, be set to one over the standard deviation of the respective impedance configuration values during a reference period. Thereby, each of the impedance configurations response to a changed physiological situation will be similar in size, i.e. each vector will affect $Z_{new}$ the same amount.

Similarly, the new admittance signal, $Y_{new}$, can be calculated as:

$$Y_{new} = offset' + \sum_{i=1}^{N} w_i' \times Y_i \cdot \frac{1}{N}$$

where $w_i'$, the weight factor, may be one over the standard deviation of the admittance values for the N vectors, respectively.

Thereafter, the short term trend parameter is calculated as a moving average or a sum over a first period of time. A preferred period of time is between about 0-5 days, another preferred period of time is about 1-3 days, a further preferred period of time is about 12 and about 48 hours, a more preferred period of time is about 18-42 hours, an even more preferred period of time is about 18-36 hours, and highly preferred period of time is about 24 hours. Further, a long trend parameter is calculated moving average or a sum over a second period of time. A preferred period of time is between about 7-75 days, another preferred period of time is about 10-45 days, a further preferred period of time is about 10-30, yet another preferred period of time is about 12-25 days, a more preferred period of time is about 12-18 days, an even more preferred period of time is about 12-16 days, and highly preferred period of time is about 14 days.

The calculated long term and short term trend parameters are supplied to a patient status determining module 47 adapted to determine a patient status index, which may be used to track the patients<'> heart failure status and/or predict acute heart failure exacerbation events in the patient, which will be described in more detail hereinafter.

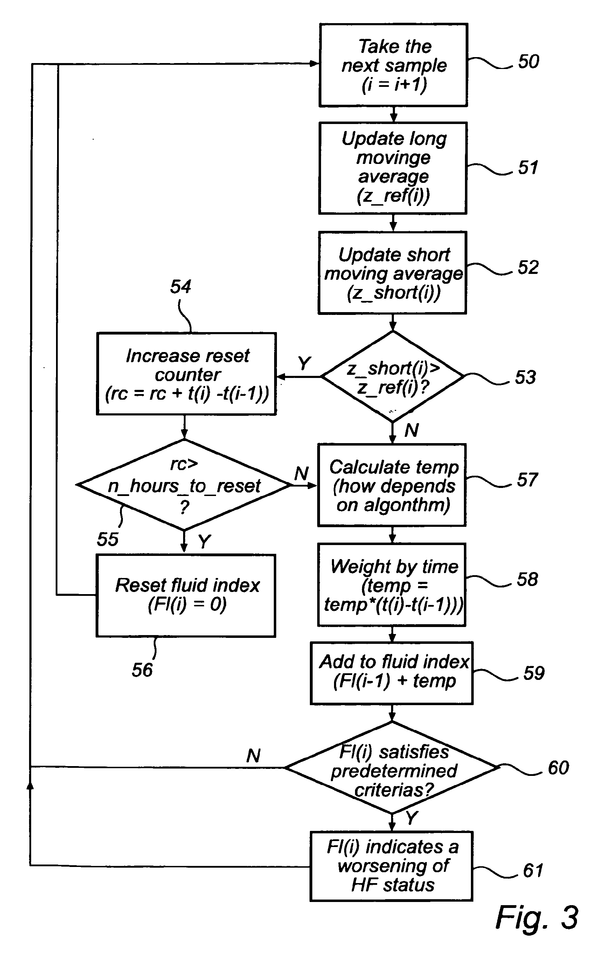

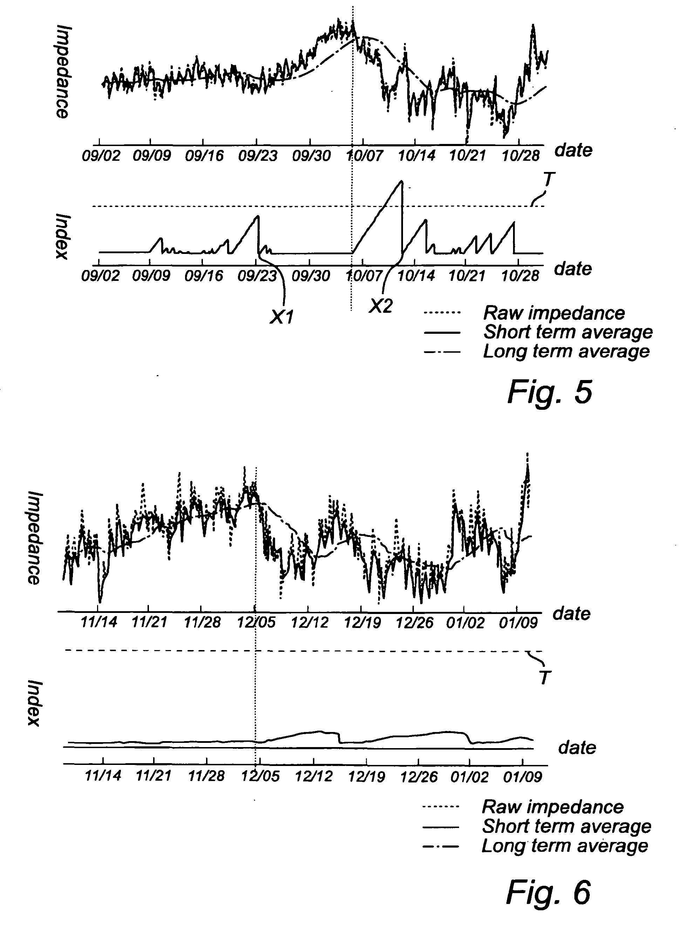

With reference to Figs. 3-6, embodiments of the present invention for determining a patient status index used for predicting a heart failure status will be discussed. In Fig. 3, a flow chart describing embodiments is illustrated, and in Figs. 5 and 6, impedance data obtained from two different patients during clinical tests are shown. Fig. 5 and 6 shows (in the upper graphs) the raw impedance, the short term average and the long term average over time for two different patients, and (in the lower graphs) the corresponding patient status index determined in accordance with different embodiments of the present invention, respectively. The dashed line T indicates a patient specific threshold, which constitutes a criteria for an indication of a worsening of a HF status in accordance with one embodiment of the present invention.

With reference now to Fig. 3, an embodiment of the method for determining a patient status index will be described. The method will be described in terms of impedance values but, as understood, a similar method may be used for admittance values. The following abbreviations are used in Fig. 3:

z_short(i) = short term average at sample i;
z_ref = reference value = long term average at sample i;
FI(i) = fluid index, or patient status index, at sample i;
t(i) = time at sample i; and re = reset counter.

First, at step 50, samples, i, are taken at predetermined time points, for example, once a day or twice a day at predetermined time points of the day, every sixth hour, every second hour, or every hour. However, as the skilled person understands, it is not necessary to use equidistant time intervals in the sampling but it is also conceivable to use intervals having irregular lengths or adapted to the interval length to the development of the patient index. For example, if the patient index has increased during a predetermined period, the sampling rate may be increased.

At step 51, the long moving average z_long(i) is updated and, at step 52, the short moving average, z_short(i), is updated as described above. Thereafter, at step 53, the long average is compared to the short average and if the short moving average is above the long moving average (i.e. the impedance is increasing), the procedure proceeds to step 54 where a reset counter is increased. Then, at step 55, it is checked whether the reset counter satisfies predetermined criterion, for example, if the reset counter has increased substantially monotonously for a predetermined number of samples, or for a predetermined period of time, for example, 0-5 days, or if the reset counter has increased above a predetermined threshold during a predetermined period of time. If the reset counter satisfies the predetermined criterion, the reset counter and the patient status index (or fluid index) are, at step 56, reset to the initial values. If the reset counter does not satisfy the criterion, the procedure returns to step 50. In Fig. 5, in which the development of the measured impedance of a patient during a period of time is shown, a reset of the index is performed, for example, at the time points shortly after 09/23 and shortly before 10/14, respectively.

However, if, at step 53, the short moving average is lower than the long moving average (i.e. the impedance is dropping or decreasing), the procedure proceeds to step 57 where a temporary value, temp, is calculated.

According to a first embodiment of the present invention, the temporary value, temp, is calculated in accordance with the following formula:

$$\text{temp} = \text{z\_ref} - \text{z\_short}.$$

According to a second embodiment of the present invention, the temporary value, temp, is calculated in accordance with the following formula:

$$temp = \frac{z_{ref}}{z_{short}} - 1.$$

Further, in accordance with a third embodiment of the present invention, the temporary value, temp, is calculated in accordance with the following formula:

$$temp = 1 - \frac{z_{short}}{z_{ref}}$$

Thereafter, at step 58, the temporary value, temp, is weighted by time to the preceding sample. Each sample may also associated with a weight corresponding to the time interval to the preceding sample. For example, if two different sample rates have been used, where the first rate is half of the second rate, the sample counts made during the first rate may be associated with the weight 1 while the sample counts made during the second rate may be given or associated with the weight ½. The weighted temporary value, $temp_{weighted}$, is determined in accordance with the following formula:

$$temp_{weighted} = q * temp * (t(i) - t(i-1)),$$

where q is the applied weight, t(i) is the time point for sample i, and t(i-1) is the time point, i-1, for the preceding sample.

Then, at step 59, the patient status index, or fluid index FI(i), is updated in accordance with the following formula:

$$FI(i) = FI(i-1) - temp.$$

Thereafter, at step 60, it is checked whether the patient status index satisfies predetermined criteria's, for example, whether the patient status index has increased substantially monotonously during a monitoring period, which may be, for example about 10-13 days, or about 11 days (264 hours) or if the patient status index has exceed a predetermined threshold. If no, the procedure returns to step 50 where the monitoring is continued. On the other hand, if yes, it is determined in step 61 that the patient index indicates an exacerbation of the patient status, and, in particular, a worsening of the HF status of the patient. In one embodiment of the present invention, the index is compared to a patient specific threshold and if the index exceeds the threshold, a warning may be issued or an alarm may be triggered. In Figs. 5 and 6, this threshold is indicated with the dashed line T. The alarm may be sent to the physician via the communication unit 49 of the stimulator 10 and an external communication system. In Fig. 4 an embodiment of such a system is disclosed. A patient 77 is a recipient of an implantable medical device, for example, a stimulator 10 in accordance with the stimulator described above. The alarm may be sent to a home monitoring device or PC 74, for example, by means of telemetered signals via an interface unit 71 such as a wand or by means of RF signals, located at the patients' home. The patient can thus be informed of the exacerbation of the heart failure status. Further, the alarm signal can be sent via an internetwork 72, such as the Internet, to a monitoring device, workstation or programmer 75 located at a health care institution for use by the nursing staff, or other authorized personnel at the institution. The workstation 75 may be a part of a server system and may be connected to a database 76 for storage of patient related information. The alarm signals are communicated over an internetwork 72, such as the Internet. However, any type of electronic communications link could be used, including an intranetwork line, serial link, data telephone link, satellite link, radio-frequency link, infrared link, fibre optic link, coaxial cable link, television link, and the like, as is known in the art.

Fig. 5 shows, in the upper graph, the impedance, the raw, the short term average and the long term average, measured for one patient over a period of time and the corresponding patient status index in the lower graph, wherein the patient status index is determined based on the temporary value calculated as:

$$temp = 1$$

Furthermore, Fig. 6 shows, in the upper graph, the impedance, the raw, the short term average and the long term average, measured for another patient over another period of time and the corresponding patient status index in the lower graph, wherein the patient status index is determined based on the temporary values:

$$temp = \frac{z_{ref}}{z_{short}} - 1,$$

or

$$temp = 1 - \frac{z_{short}}{z_{ref}}$$

The programmable threshold will depend on which algorithm is used for determining the patient status index and will also be different between patients. Since the impedance is affected not only by the heart failure status but also by lead location, blood constituents, if the patient is on dialysis, diet, exercise, posture, etc, the normal impedance (and hence index) variability may be different from patient to patient.

The implantable medical device or stimulator 10 according to the present invention, may include further sensors, for example, posture sensors, blood sensors, and wherein information from these additional sensors may be used to increase the accuracy of the impedance measurements.

Although an exemplary embodiment of the present invention has been shown and described, it will be apparent to those having ordinary skill in the art that a number of changes, modifications, or alterations to the inventions as described herein may be made. Thus, it is to be understood that the above description of the invention and the accompanying drawings is to be regarded as a non-limiting.

## Claims

1. An implantable medical device (10) for monitoring a patient status, comprising:

   an impedance circuit (41) adapted to acquire impedance signals during consecutive impedance measuring sessions;
   an impedance processing module (46) adapted to process said impedance signals to determine a first trend parameter based on multiple impedance signals measured over a first period of time and to determine a second trend parameter based on multiple impedance signals measured over a second period of time, which second period of time is significantly longer than said first period of time; and
   a patient status determining module (47) adapted to:

   determine a patient status index, wherein said first trend parameter and said second trend parameter are compared at predetermined sample points of time ; and
   determine a patient status based on said patient status index, wherein an indication of an exacerbation of said patient status is determined if predetermined conditions are satisfied, **characterized in that** said patient status index is increased based on counted samples where said first trend parameter is lower than said second trend parameter, determine said second trend parameter based on multiple impedance signals and said predetermined conditions including that a patient status index has increased substantially monotonously during a first monitoring period.

2. The implantable medical device according to claim 1, wherein said impedance processing module (46) is adapted to calculate each impedance value used to determine said first and/or second trend parameters as a composite value of impedance values acquired by means of at least two impedance measurement vector configurations, wherein each impedance configuration is associated with a predetermined weight factor.

3. The implantable medical device according to claim 1 or 2, wherein said patient status determining module (47) is adapted to:

   count samples, when impedance trend parameters are used, at which said first trend parameter is lower or above than said second trend parameter, wherein said index is increased at a counted sample when the first trend parameter is lower than said second trend parameter and decreased when said first trend parameter is above said second parameter,
   count samples, when admittance trend parameters are used, at which said first trend parameter is higher or lower than said second trend parameter, and wherein said patient status index corresponds to said counts, wherein said index is decreased at a counted sample when the first trend parameter is lower than said second trend parameter and increased when said first trend parameter is above said second parameter; and
   determine a patient status based on said patient status index, wherein said predetermined conditions includes that a patient status index that has increased substantially monotonously during a first monitoring period is determined to be an indication of an exacerbation of patient status.

4. The implantable medical device according to anyone of preceding claims, wherein said predetermined conditions are patient specific, said patient specific conditions being, manually and/or automatically, adaptable.

5. The implantable medical device according to any one of preceding claims, wherein a situation where said patient status index has increased such that a predetermined threshold has been exceeded during said first monitoring period is determined to be an indication of an exacerbation of said patient status.

6. The implantable medical device according to any one of preceding claims, wherein said patient status determining module (47) is adapted to:

   count samples, when impedance trend parameters are used, at which said first trend parameter is higher than said second trend parameter, or to count samples, when admittance trend parameters are used, at which said first trend parameter is lower than said second trend parameter;
   determine a reset measure based on said counts; and

reset said patient status index to an initial value if said reset measure has increased substantially monotonously during a second monitoring period, said second monitoring period being substantially shorter than said first monitoring period.

**7.** The implantable medical device according to claim 6, wherein said patient status index is reset to said initial value if said reset measure has increased such that a predetermined reset threshold has been exceeded during said second monitoring period.

**8.** The implantable medical device according to any one of preceding claims, wherein said patient status determining module (47) is adapted to:

> determine a temporary value reflecting a ratio between said first trend parameter and said second trend parameter; and
> if, when impedance trend parameters are used, said temporary value is below a predetermined value or if, when admittance trend parameters are used, said temporary value above a predetermined value, determine a sum for a present sample to be equal to the sum at a preceding sample and said temporary value multiplied with the period of time lapsed since said preceding sample, wherein said patient status index is determined to correspond to said sum for said present sample.

**9.** The implantable medical device according to claim 8, wherein said patient status determining module (47) is adapted to:

> determine said temporary value as being equal to the quote between said first trend parameter and said second trend parameter subtracted with a value corresponding to 1; and
> rectify said temporary value.

**10.** The implantable medical device according to any one of preceding claims 1-7, wherein said patient status determining module (47) is adapted to:

> determine a temporary value reflecting a ratio between said second trend parameter and said first trend parameter; and
> if said temporary value exceeds a predetermined value when impedance trend parameters are used or below said predetermined value when admittance trend parameters are used, determine a sum for a present sample to be equal to the sum at a preceding sample and said ratio multiplied with the period of time elapsed since said preceding sample, wherein said patient status index is determined to correspond to said sum for said present sample.

**11.** The implantable medical device according to claim 10, wherein said patient status determining module (47) is adapted to:

> determine said temporary value as being equal to the quote between said second trend parameter and said first trend parameter subtracted with a value corresponding to 1.

**12.** The implantable medical device according to any one of preceding claims, wherein said patient status determining module (47) is adapted to:

> compare said patient status index with a predetermined patient specific threshold; and
> if said patient status index exceeds said patient specific threshold, issue a warning signal that triggers an alarm function.

**Patentansprüche**

**1.** Implantierbare medizinische Vorrichtung (10) zum Überwachen eines Patientenstatus, umfassend:

> eine Impedanzschaltung (41), die ausgelegt ist, Impedanzsignale während aufeinanderfolgender Impedanz-Messsitzungen aufzunehmen;
> ein Impedanz-Verarbeitungsmodul (46), das ausgelegt ist, die Impedanzsignale zu verarbeiten, um einen ersten Trendparameter auf Grundlage mehrfacher Impedanzsignale zu bestimmen, die über eine erste Zeitperiode gemessen werden, und um einen zweiten Trendparameter auf Grundlage mehrfacher Impedanzsignale zu bestimmen, die über eine zweite Zeitperiode gemessen werden, wobei die zweite Zeitperiode beträchtlich länger als die erste Zeitperiode ist, und
> ein Patientenstatus-Bestimmungsmodul (47), das ausgelegt ist:

>> einen Patientenstatusindex zu bestimmen, wobei der erste Trendparameter und der zweite Trendparameter bei vorbestimmten Zeitabtastpunkten verglichen werden; und
>> einen Patientenstatus auf Grundlage des Patientenstatusindex zu bestimmen, wobei eine Anzeige einer Verschlimmerung des Patientenstatus bestimmt wird, wenn vorbestimmte Bedingungen erfüllt sind, **dadurch gekennzeichnet, dass** der Patientenstatusindex auf Grundlage gezählter Abtastwerte erhöht wird, wobei der erste Trendparameter niedriger als der zweite Trendparameter ist,

den zweiten Trendparameter auf Grundlage mehrfacher Impedanzsignale und der vorbestimmten Bedingungen zu bestimmen, die einschließen, dass ein Patientenstatusindex im Wesentlichen monoton während einer ersten Überwachungsperiode zugenommen hat.

2. Implantierbare medizinische Vorrichtung nach Anspruch 1, wobei das Impedanz-Verarbeitungsmodul (46) ausgelegt ist, jeden Impedanzwert zu berechnen, der verwendet wird, um die ersten und/oder zweiten Trendparameter zu bestimmen, als einen zusammengesetzten Wert von Impedanzwerten, die mittels zumindest zwei Impedanzmess-Verktorkonfigurationen erfasst werden, wobei jeder Impedanzkonfiguration ein vorbestimmter Gewichtsfaktor zugeordnet ist.

3. Implantierbare medizinische Vorrichtung nach Anspruch 1 oder 2, wobei das Patientenstatus-Bestimmungsmodul (47) ausgelegt ist:

Abtastwerte zu zählen, wenn Impedanz-Trendparameter verwendet werden, bei welchen der erste Trendparameter niedriger als der oder oberhalb des zweiten Trendparameters ist, wobei der Index bei einem gezählten Abtastwert erhöht wird, wenn der erste Trendparameter niedriger als der zweite Trendparameter ist, und verringert wird, wenn der erste Trendparameter oberhalb des zweiten Trendparameters ist, Abtastwerte zu zählen, wenn Admittanz-Trendparameter verwendet werden, bei welchen der erste Trendparameter höher oder niedriger als der zweite Trendparameter ist, und wobei der Patientenstatusindex den Zählwerten entspricht, wobei der Index bei einem gezählten Abtastwert verringert wird, wenn der erste Trendparameter niedriger als der zweite Trendparameter ist, und erhöht wird, wenn der erste Trendparameter oberhalb des zweiten Trendparameters ist; und einen Patientenstatus auf Grundlage des Patientenstatusindex zu bestimmen, wobei die vorbestimmten Bedingungen einschließen, dass ein Patientenstatusindex, der im Wesentlichen monoton während einer ersten Überwachungsperiode zugenommen hat, bestimmt wird, eine Anzeige einer Verschlimmerung eines Patientenstatus zu sein.

4. Implantierbare medizinische Vorrichtung nach einem der voranstehenden Ansprüche, wobei die vorbestimmten Bedingungen patientenspezifisch sind, wobei die patientenspezifischen Bedingungen manuell und/oder automatisch anpassbar sind.

5. Implantierbare medizinische Vorrichtung nach einem der voranstehenden Ansprüche, wobei eine Situation, bei der der Patientenstatusindex derart zugenommen hat, dass eine vorbestimmte Schwelle während der ersten Überwachungsperiode überschritten worden ist, bestimmt wird, eine Anzeige einer Verschlimmerung des Patientenstatus zu sein.

6. Implantierbare medizinische Vorrichtung nach einem der voranstehenden Ansprüche, wobei das Patientenstatus-Bestimmungsmodul (47) ausgelegt ist:

Abtastwerte zu zählen, wenn Impedanz-Trendparameter verwendet werden, bei welchen der erste Trendparameter höher als der zweite Trendparameter ist, oder Abtastwerte zu zählen, wenn Admittanz-Trendparameter verwendet werden, bei welchen der erste Trendparameter niedriger als der zweite Trendparameter ist; ein Rücksetzmaß auf Grundlage der Zählwerte zu bestimmen; und den Patientenstatusindex auf einen Anfangswert zurückzusetzen, wenn das Rücksetzmaß im Wesentlichen monoton während einer zweiten Überwachungsperiode zugenommen hat, wobei die zweite Überwachungsperiode beträchtlich kürzer als die erste Überwachungsperiode ist.

7. Implantierbare medizinische Vorrichtung nach Anspruch 6, wobei der Patientenstatusindex auf den Anfangswert zurückgesetzt wird, wenn das Rücksetzmaß derart zugenommen hat, dass eine vorbestimmte Rücksetzschwelle während der zweiten Überwachungsperiode überschritten worden ist.

8. Implantierbare medizinische Vorrichtung nach einem der voranstehenden Ansprüche, wobei das Patientenstatus-Bestimmungsmodul (47) ausgelegt ist:

einen vorübergehenden Wert, der ein Verhältnis zwischen dem ersten Trendparameter und dem zweiten Trendparameter widerspiegelt, zu bestimmen; und falls, wenn Impedanz-Trendparameter verwendet werden, der vorübergehende Wert unterhalb eines vorbestimmten Werts ist, oder falls, wenn Admittanz-Trendparameter verwendet werden, der vorübergehende Wert oberhalb eines vorbestimmten Werts ist, eine Summe für einen gegenwärtigen Abtastwert zu bestimmen, gleich der Summe bei einem vorangehenden Abtastwert und dem vorübergehenden Wert multipliziert mit der Zeitperiode zu sein, die nach dem vorangehenden Abtastwert verstrichen ist, wobei der Patientenstatusindex bestimmt wird, der Summe für den gegenwärtigen Abtastwert zu entsprechen.

9. Implantierbare medizinische Vorrichtung nach Anspruch 8, wobei das Patientenstatus-Bestimmungsmodul (47) ausgelegt ist:

den vorübergehenden Wert zu bestimmen, gleich der Quote zwischen dem ersten Trendparameter und dem zweiten Trendparameter, subtrahiert mit einem Wert, der 1 entspricht, zu sein; und
den vorübergehenden Wert gleichzurichten.

10. Implantierbare medizinische Vorrichtung nach einem der voranstehenden Ansprüche 1 bis 7, wobei das Patientenstatus-Bestimmungsmodul (47) ausgelegt ist:

einen vorübergehenden Wert, der ein Verhältnis zwischen dem zweiten Trendparameter und dem ersten Trendparameter widerspiegelt, zu bestimmen; und
wenn der vorübergehende Wert einen vorbestimmten Wert überschreitet, wenn Impedanz-Trendparameter verwendet werden, oder unter dem vorbestimmten Wert ist, wenn Admittanz-Trendparameter verwendet werden, eine Summe für einen gegenwärtigen Abtastwert zu bestimmen, gleich der Summe bei einem vorangehenden Abtastwert und dem Verhältnis, multipliziert mit der Zeitperiode zu sein, die seit dem vorangehenden Abtastwert verstrichen ist, wobei der Patientenstatusindex bestimmt ist, der Summe für den gegenwärtigen Abtastwert zu entsprechen.

11. Implantierbare medizinische Vorrichtung nach Anspruch 10, wobei das Patientenstatus-Bestimmungsmodul (47) ausgelegt ist:

den vorübergehenden Wert zu bestimmen, gleich der Quote zwischen dem zweiten Trendparameter und dem ersten Trendparameter, subtrahiert mit einem Wert, der 1 entspricht, zu sein.

12. Implantierbare medizinische Vorrichtung nach einem der voranstehenden Ansprüche, wobei das Patientenstatus-Bestimmungsmodul (47) ausgelegt ist:

den Patientenstatusindex mit einer vorbestimmten patientenspezifischen Schwelle zu vergleichen; und
wenn der Patientenstatusindex die patientenspezifische Schwelle überschreitet, ein Warnsignal auszugeben, das eine Alarmfunktion auslöst.

**Revendications**

1. Dispositif médical implantable (10) destiné à surveiller l'état d'un patient, comprenant :

un circuit d'impédance (41) adapté pour acquérir des signaux d'impédance pendant des séances de mesure d'impédance consécutives ;
un module d'exploitation de l'impédance (46) adapté pour traiter lesdits signaux d'impédance pour déterminer un premier paramètre de tendance sur la base de multiples signaux d'impédance mesurés pendant une première période de temps et pour déterminer un second paramètre de tendance sur la base de multiples signaux d'impédance mesurés pendant une seconde période de temps, laquelle seconde période de temps étant sensiblement plus longue que ladite première période de temps et
un module de détermination de l'état du patient (47), adapté pour :

déterminer un indice d'état du patient, ledit premier paramètre de tendance et ledit second paramètre de tendance étant comparés à des temps prédéterminés d'échantillonnage, et
déterminer un état du patient sur la base dudit indice d'état du patient, une indication d'une aggravation dudit état du patient étant déterminée si des conditions prédéterminées sont remplies, **caractérisé en ce que** ledit indice d'état du patient est augmenté sur la base d'échantillons comptés pour lesquels ledit premier paramètre de tendance est inférieur audit second paramètre de tendance,
déterminer ledit second paramètre de tendance sur la base de multiples signaux d'impédance et lesdites conditions prédéterminées comprenant le fait qu'un indice d'état du patient ait augmenté sensiblement de manière monotone pendant une première période de surveillance.

2. Dispositif médical implantable selon la revendication 1, dans lequel ledit module d'exploitation de l'impédance (46) est adapté pour calculer chaque valeur d'impédance utilisée pour déterminer lesdits premier et/ou second paramètres de tendance en tant que valeur composée de valeurs d'impédance acquises au moyen d'au moins deux configurations de vecteur de mesure d'impédance, chaque configuration d'impédance étant associée à un coefficient de pondération prédéterminé.

**3.** Dispositif médical implantable selon la revendication 1 ou 2, dans lequel ledit module de détermination de l'état du patient (47) est adapté pour :

quand des paramètres de tendance d'impédance sont utilisés, compter les échantillons pour lesquels ledit premier paramètre de tendance est inférieur ou supérieur audit second paramètre de tendance, ledit indice étant augmenté lorsqu'un échantillon est compté quand le premier paramètre de tendance est inférieur audit second paramètre de tendance et diminué quand ledit premier paramètre de tendance est supérieur audit second paramètre,

quand des paramètres de tendance d'admittance sont utilisés, compter les échantillons pour lesquels ledit premier paramètre de tendance est supérieur ou inférieur audit second paramètre de tendance, et ledit indice d'état du patient correspondant audit nombre d'échantillons comptés, ledit indice étant diminué lorsqu'un échantillon est compté quand le premier paramètre de tendance est inférieur audit second paramètre de tendance et augmenté quand ledit premier paramètre de tendance est supérieur audit second paramètre ; et

déterminer un état du patient sur la base dudit indice d'état du patient, lesdites conditions prédéterminées comprenant le fait qu'un indice d'état du patient ayant augmenté sensiblement de manière monotone pendant une première période de surveillance soit déterminé comme étant une indication d'une aggravation de l'état du patient.

**4.** Dispositif médical implantable selon l'une quelconque des revendications précédentes, dans lequel lesdites conditions prédéterminées sont spécifiques au patient, lesdites conditions spécifiques au patient pouvant être adaptées, manuellement et/ou automatiquement.

**5.** Dispositif médical implantable selon l'une quelconque des revendications précédentes, dans lequel une situation dans laquelle ledit indice d'état du patient a tellement augmenté qu'une valeur seuil prédéterminée a été dépassée pendant ladite première période de surveillance est déterminée comme étant une indication d'une aggravation dudit état du patient.

**6.** Dispositif médical implantable selon l'une quelconque des revendications précédentes, dans lequel ledit module de détermination de l'état du patient (47) est adapté pour :

quand des paramètres de tendance d'impédance sont utilisés, compter les échantillons pour lesquels ledit premier paramètre de tendance est supérieur audit second paramètre de tendance, ou, quand des paramètres de tendance d'admittance sont utilisés, pour compter les échantillons pour lesquels ledit premier paramètre de tendance est inférieur audit second paramètre de tendance ;

déterminer une mesure de remise à l'état initial sur la base dudit nombre d'échantillons comptés ; et

remettre ledit indice d'état du patient à une valeur initiale si ladite mesure de remise à l'état initial a augmenté sensiblement de manière monotone pendant une seconde période de surveillance, ladite seconde période de surveillance étant sensiblement plus courte que ladite première période de surveillance.

**7.** Dispositif médical implantable selon la revendication 6, dans lequel ledit indice d'état du patient est remis à ladite valeur initiale si ladite mesure de remise à l'état initial a tellement augmenté qu'une valeur seuil de remise à l'état initial prédéterminée a été dépassée pendant ladite seconde période de surveillance.

**8.** Dispositif médical implantable selon l'une quelconque des revendications précédentes, dans lequel ledit module de détermination de l'état du patient (47) est adapté pour :

déterminer une valeur temporaire reflétant un rapport entre ledit premier paramètre de tendance et ledit second paramètre de tendance ; et

si, quand des paramètres de tendance d'impédance sont utilisés, ladite valeur temporaire est inférieure à une valeur prédéterminée ou si, quand des paramètres de tendance d'admittance sont utilisés, ladite valeur temporaire est supérieure à une valeur prédéterminée, déterminer une somme pour un échantillon présent pour qu'elle soit égale à la somme lors d'un échantillon précédent et ladite valeur temporaire multipliée par la période de temps s'étant écoulée depuis ledit échantillon précédent, ledit indice d'état du patient étant déterminé comme correspondant à ladite somme pour ledit échantillon présent.

**9.** Dispositif médical implantable selon la revendication 8, dans lequel ledit module de détermination de l'état du patient (47) est adapté pour :

déterminer ladite valeur temporaire comme étant égale au quotient entre ledit premier paramètre de tendance et ledit second paramètre de tendance auquel est soustraite une valeur correspondant à 1 ; et

rectifier ladite valeur temporaire.

**10.** Dispositif médical implantable selon l'une quelconque des revendications précédentes 1 à 7, dans lequel ledit module de détermination de l'état du patient (47) est adapté pour :

déterminer une valeur temporaire reflétant un rapport entre ledit second paramètre de tendance et ledit premier paramètre de tendance ; et si ladite valeur temporaire dépasse une valeur prédéterminée quand des paramètres de tendance d'impédance sont utilisés ou est inférieure à ladite valeur prédéterminée quand des paramètres de tendance d'admittance sont utilisés, déterminer une somme pour un échantillon présent pour qu'elle soit égale à la somme lors d'un échantillon précédent et ledit rapport multiplié par la période de temps s'étant écoulée depuis ledit échantillon précédent, ledit indice d'état du patient étant déterminé comme correspondant à ladite somme pour ledit échantillon présent.

**11.** Dispositif médical implantable selon la revendication 10, dans lequel ledit module de détermination de l'état du patient (47) est adapté pour :

déterminer ladite valeur temporaire comme étant égale au quotient entre ledit second paramètre de tendance et ledit premier paramètre de tendance auquel est soustraite une valeur correspondant à 1.

**12.** Dispositif médical implantable selon l'une quelconque des revendications précédentes, dans lequel ledit module de détermination de l'état du patient (47) est adapté pour :

comparer ledit indice d'état du patient avec une valeur seuil spécifique au patient prédéterminée ; et
si ledit indice d'état du patient dépasse ladite valeur seuil spécifique au patient, émettre un signal d'avertissement qui déclenche une fonction d'alarme.

*Fig. 1*

Fig. 2

**Fig. 3**

Flowchart contents:

- 50: Take the next sample (i = i+1)
- 51: Update long movinge average (z_ref(i))
- 52: Update short moving average (z_short(i))
- 53: z_short(i) > z_ref(i)?
- 54: Increase reset counter (rc = rc + t(i) -t(i-1))
- 55: rc > n_hours_to_reset ?
- 56: Reset fluid index (FI(i) = 0)
- 57: Calculate temp (how depends on algorithm)
- 58: Weight by time (temp = temp*(t(i)-t(i-1)))
- 59: Add to fluid index (FI(i-1) + temp
- 60: FI(i) satisfies predetermined criterias?
- 61: FI(i) indicates a worsening of HF status

Fig. 4

Fig. 5

Fig. 6

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20080024293 A
- US 20060276848 A
- US 7272443 B